# DEMANDE DE BREVET EUROPEEN

(11) **EP 2 158 865 A1**
(43) Date de publication de la demande: **03.03.2010**
(21) Numéro de dépôt: 09290640.3
(22) Date de dépôt: 25.08.2009
(51) Int. Cl.: A61B 17/86, A61B 17/88

(54) **Système de fixation externe pour réaliser une ostéosynthèse entre deux portions d'os**

(30) Priorité: 01.09.2008 FR 0804776
(71) Demandeur: Renard, Xavier, 91430 Vauhallan (FR)
(72) Inventeur: Kapandji, Adalbert, Gravigny, 91160 Longjumeau (FR); Hoel, Gérard, 97400 Saint-Denis, Ile-de-la-Réunion (FR); Renard, Xavier, 91430 Vauhallan (FR)
(74) Mandataire: Flavenot, Bernard

(57) **Abrégé**

La présente invention concerne les systèmes de fixation externes pour une ostéosynthèse entre deux portions d'os O₁, O₂ d'un os long d'axe 1 après une fracture F_{rc} définissant une ligne de fracture L_{fr} sur la surface externe de l'os.

Le système selon l'invention est caractérisé par le fait qu'il comporte une broche 10 implantable par son extrémité 11 dans la corticale de la portion O₁, cette broche pénétrant par la ligne de fracture en faisant un angle α par rapport à l'axe 1 de façon que son extrémité 12 émerge de la fracture F_{rc} en regard de la portion O₂, cette extrémité 12 faisant avec l'extrémité 11, un angle β, une broche 20 implantable dans la corticale de la portion O₂ par son extrémité 21, son extrémité 22 émergeant de cette portion O₂, des moyens 30 pour guider, par rapport à l'extrémité 12, l'implantation de l'extrémité 21 de la broche dans la corticale de la portion O₂, et des moyens 40 pour solidariser l'extrémité 12 avec l'extrémité 22.

Application, notamment, à la solidification d'une fracture extra articulaire simple de l'extrémité distale d'un radius.

## Description

La présente invention concerne les systèmes de fixation externes pour la réalisation d'une ostéosynthèse entre deux portions d'un os qui peut être considéré comme un os long définissant un axe longitudinal, les deux portions d'os étant séparées par une fracture, ces systèmes trouvant une application particulièrement avantageuse à la réduction et la solidification par ostéosynthèse d'une fracture se situant à l'extrémité distale d'un radius, quand cette fracture est du type extra articulaire simple.

Il est connu des systèmes souvent désignés sous le terme générique de "fixateurs externes", qui permettent de réaliser une ostéosynthèse entre deux portions d'os, par exemple après une fracture. D'une façon très générale, ils sont constitués par au moins deux broches qui sont implantées respectivement dans chacune des deux portions d'os, de part d'autre de la fracture, et par des moyens pour maintenir ces deux broches à distance, avec éventuellement en outre des moyens pour appliquer, entre ces deux broches, de façon permanente ou pas, des forces, par exemple élastiques, de distraction ou de traction selon le souhait du Praticien en fonction des solutions que son diagnostic lui a permis de retenir pour la réduction et la solidification de la fracture.

De tels systèmes sont par exemple décrits dans les FR-A-2912895 et 1462646, les WO 01/12081, 01/93768 et 05/084568, et le US-A-2008/021474.

Ces systèmes définis ci-dessus donnent de bons résultats pour des fractures classiques et qui concernent le plus souvent, mais non exclusivement, des os longs, en tout cas ayant une structure qui permette l'implantation d'un tel fixateur. Ce n'est pas toujours le cas, par exemple pour une fracture de l'extrémité distale d'un radius qui survient très souvent chez les personnes âgées dont la dureté des os se dégrade, notamment à cause de l'ostéoporose.

De plus, ces systèmes sont relativement complexes, lourds et encombrants. Ils gênent le patient et exigent en outre du Praticien un travail d'implantation non négligeable.

Aussi, la présente invention a-t-elle pour but de réaliser un système de fixation externe pour la réalisation d'une ostéosynthèse entre deux première et seconde portions d'un os long définissant un axe longitudinal séparées par une fracture, qui pallie au moins en grande partie les inconvénients mentionnés ci-dessus des fixateurs externes selon l'art antérieur.

Plus précisément, la présente invention a pour objet un système de fixation externe pour la réalisation d'une ostéosynthèse entre deux première et seconde portions d'un os, notamment d'un os long définissant un axe longitudinal, les deux première et seconde portions d'os étant séparées par une fracture définissant une ligne de fracture sur la surface externe de l'os, tel que défini dans la revendication 1 annexée.

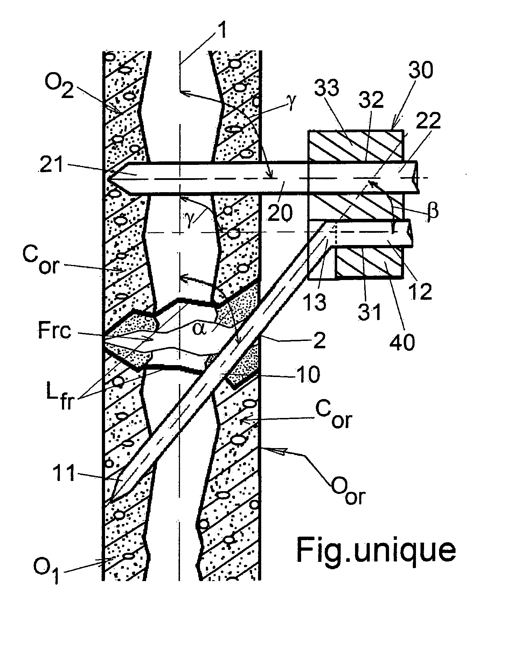

D'autres caractéristiques et avantages de l'invention apparaîtront au cours de la description suivante donnée en regard du dessin annexé à titre illustratif mais nullement limitatif, dans lequel la figure unique représente, vu en coupe schématique, le principe du système de fixation externe selon l'invention pour réaliser une ostéosynthèse entre deux première et seconde portions d'un os.

En référence à la figure unique, la présente invention concerne un système de fixation externe pour la réalisation d'une ostéosynthèse entre deux première et seconde portions O₁, O₂ d'un os Oₒᵣ, notamment d'un os long, par exemple un radius ou analogue, définissant un axe longitudinal 1, les deux première et seconde portions d'os O₁, O₂ étant séparées par une fracture F_{rc} définissant une ligne de fracture L_{fr} sur la surface externe de l'os Oₒᵣ Ce système trouve une application plus particulièrement avantageuse à la réduction et l'ostéosynthèse d'une fracture extra articulaire simple de l'extrémité distale d'un radius, notamment mais pas exclusivement chez les personnes âgées.

II est à souligner que, sur la figure unique, les deux portions d'os O₁, O₂ sont représentées après avoir été distractées du fait de la présence d'une broche selon l'invention, comme il sera explicité ci-après. De cette façon les deux portions d'os sont éloignées l'une de l'autre et la ligne de fracture est décomposée en deux lignes non confondues. Il est cependant précisé que le système selon l'invention, peut s'appliquer aussi bien à une facture dont les deux bords opposés sont au contact, qu'à une fracture qui a ses deux bords non au contact.

En effet, dans le cas d'une telle fracture, il arrive très souvent qu'il se produise, en plus de la fracture, un tassement de la partie osseuse fracturée, notamment à cause de l'ostéoporose chez les personnes âgées.

Pour réduire (i) et consolider (ii) une telle fracture, il est donc nécessaire de procéder généralement, tout d'abord à une distraction {première fonction (i)} entre les deux portions d'os séparées par la fracture afin de les écarter pour qu'elles reprennent le plus possible leur position relative originelle, puis à réaliser l'ostéosynthèse {seconde fonction (ii)}.

Comme mentionné au préambule de la présente description, il existe des fixateurs qui peuvent réaliser les deux fonctions définies ci-dessus (i) et (ii), mais ils sont complexes, très encombrants et lourds, relativement encore plus pour les personnes âgées.

Le système selon l'invention permet de réaliser ces deux fonctions, tout en ayant une structure très simple, légère et peut encombrante.

Comme illustré sur la figure unique représentant le principe de sa réalisation préférée, le système comporte au moins un premier ensemble comprenant au moins une première broche 10 apte à être implantée par son extrémité distale 11 dans la partie corticale Cₒᵣ de la première portion d'os O₁ opposée à un point 2 de la ligne de fracture L_{fr}, cette première broche étant apte à pénétrer dans l'os Oₒᵣ par cette ligne de fracture en ce point 2 en faisant, avec l'axe longitudinal 1, un angle α différent de 90° et de façon que son extrémité proximale émerge de la fracture F_{rc} par la ligne de fracture L_{fr}.

Le système comporte aussi une seconde broche rectiligne 20 apte à être implantée dans la partie corticale Cₒᵣ de la seconde portion d'os O₂ par son extrémité distale 21 et de façon que son extrémité proximale 22 émerge de cette seconde portion d'os O₂.

Le système comporte en outre des moyens 40 pour solidariser l'extrémité proximale émergente 12 de la première broche 10 et l'extrémité proximale émergente 22 de la seconde broche 20 quand ces deux broches ont été implantées.

Selon une réalisation avantageuse, l'extrémité proximale 12 de la première broche 10 est réalisée de façon qu'elle émerge de la fracture F_{rc} pour se positionner en regard de la seconde portion d'os O₂, et que cette extrémité proximale émergente 12 fasse, d'une part avec l'extrémité distale 11 un coude 13 d'angle β non nul, et d'autre part avec l'axe longitudinal 1 un angle γ non nul égal à α + β. (Remarque : les angles sont référencés par rapport à leur représentation sur la figure, pour éviter de les définir avec un sens d'orientation mathématique et/ou trigonométrique dans l'unique souci de simplifier la présente description.).

Le système comporte aussi, de façon très avantageuse notamment pour faciliter l'implantation de la seconde broche 20, des moyens 30 pour guider, par rapport à l'extrémité proximale émergente 12 de la première broche 10, l'implantation de l'extrémité distale 21 de la seconde broche 20 dans la partie corticale de la seconde portion d'os O₂.

Selon une réalisation préférentielle, ces moyens 30 pour guider l'implantation de l'extrémité distale 21 de la seconde broche 20 sont agencés de façon que cette seconde broche 20 fasse, avec l'axe longitudinal 1, un angle sensiblement. égal à l'angle γ défini ci-avant et, de préférence, de façon que les deux extrémités proximales émergentes 12, 22 des première et seconde broches 10, 20 soient sensiblement parallèles.

Toujours selon un mode de réalisation préférentiel, les moyens 30 pour guider l'implantation de l'extrémité distale de la seconde broche 20 sont constitués, comme illustré sur la figure unique, d'une pièce guide 33 dans laquelle sont réalisées deux percées traversantes 31, 32 ou analogues ayant des sections transversales respectivement complémentaires de celles des extrémités proximales émergentes 12, 22 des première et seconde broches 10, 20. En conséquence, quand ces deux extrémités proximales émergentes 12, 22, les axes des deux percées traversantes 31, 32 sont sensiblement parallèles.

Quant aux moyens 40 pour solidariser l'extrémité proximale émergente 12 de la première broche 10 et l'extrémité proximale émergente 22 de la seconde broche rectiligne 20, ils sont avantageusement constitués par le fait que la pièce guide 33, définie ci-dessus, est réalisée en un matériau, ou à base d'un matériau, apte à être déformé par exemple plastiquement de façon qu'elle soit apte à être sertie sur les deux extrémités proximales émergentes 12, 22 des première et seconde broches 10, 20 quand celles-ci sont respectivement positionnées dans les percées traversantes 31, 32, afin de figer la distance entre les deux portions d'os O₁ O₂ pour obtenir l'ostéosynthèse. Un tel matériau est par exemple de l'acier recuit ou analogue.

Comme illustré sur la figure unique, il est en outre avantageux que la pièce guide 33 englobe au moins une partie du coude 13 défini auparavant, de façon à éviter que la première broche 10 ne puisse, sous des efforts de quelque origine qu'ils soient, tourner dans sa percée correspondante 31. Mais il est aussi possible, pour éviter ce pivotement, que les extrémités proximales 12, 22 des deux broches 10, 20 qui seront serties aient des sections polygonales, par exemple carrées ou analogues.

Avec ce système selon l'invention, il est possible de réaliser les deux fonctions définies ci-avant: d'une part, en étant introduite entre les deux portions d'os O₁, O₂, la première broche 10 les éloigne à la manière d'un coin et réalise ainsi la distraction entre ces deux portions d'os {(fonction (i)}, et d'autre part, la fixation solidaire des deux broches 10, 20 entre elles, par les moyens 40, permet de maintenir les deux portions d'os O₁, O₂ à la distance voulue et réaliser ainsi l'ostéosynthèse entre elles {(fonction (ii)}.

Toujours selon un mode de réalisation préférentiel, le système peut comporter en outre des moyens pour tordre la première broche 10, qui est toujours disponible dans le commerce sous forme rectiligne, de façon que son extrémité proximale émergente 12 forme avec son extrémité distale 11 le coude 13 d'angle β.

Selon une réalisation possible ces moyens pour tordre la première broche 10, à l'origine rectiligne, et former le coude 13 comportent un rapporteur et une pince de pliage.

Selon une réalisation possible, le rapporteur peut comporter un élément apte à se fixer sur la première broche 10 avant qu'elle ne soit tordue et sur lequel est apte à être repérée la valeur de l'angle β, de façon à pouvoir tordre cette première broche, par exemple d'abord dans n'importe quelle position de celle-ci par rapport à la première portion d'os O₁, puis à la ramener dans sa position déterminée de façon que sa partie proximale émergente 12 fasse avec sa partie distale l'angle β déterminé par le Praticien.

Dans le mode de réalisation décrit ci-dessus et illustré sur la figure unique, le système selon l'invention ne comporte qu'un ensemble comprenant un couple des deux broches 10, 20. Cependant, il est possible, et même avantageux dans la plupart des cas, qu'il comporte au moins un second ensemble comprenant un autre couple de première et seconde broches, les première et seconde broches de ce second ensemble étant non colinéaires par rapport aux première et seconde broches 10, 20 du premier ensemble, et les deux points 2 de la ligne de fracture L_{fr} pour les deux ensembles étant bien entendu non confondus.

Selon une mise en oeuvre en accord avec la représentation de la figure unique, qui donne de bons résultats, l'angle α est sensiblement égal à 45°, ainsi que l'angle β, de façon que l'angle γ soit sensiblement égal à 90°.

A la description faite ci-dessus, il apparaît à l'évidence que les buts de la présente invention sont sensiblement atteints, à savoir la réalisation d'un système pour réaliser une ostéosynthèse entre deux portions d'un os, qui est d'une structure très simple, facile à mettre en place et peu encombrante.

## Revendications

1. Système de fixation externe pour la réalisation d'une ostéosynthèse entre deux première et seconde portions d'un os (O₁, O₂), notamment d'un os long définissant un axe longitudinal (1), les deux première et seconde portions d'os étant séparées par une fracture (F_{rc}) définissant une ligne de fracture sur la surface externe de l'os, **caractérisé par le fait qu'**il comporte au moins un premier ensemble comprenant au moins :
• une première broche (10) apte à être implantée par son extrémité distale (11) dans la partie corticale de la première portion d'os (O₁) opposée à un point (2) de la ligne de fracture, ladite première broche étant apte à pénétrer dans l'os par cette ligne de fracture en ce point (2) en faisant, avec l'axe longitudinal (1), un angle α différent de 90° et de façon que son extrémité proximale émerge de ladite fracture (F_{rc}),
• une seconde broche rectiligne (20) apte à être implantée dans la partie corticale de la seconde portion d'os (O₂) par son extrémité distale (21) et de façon que son extrémité proximale (22) émerge de cette seconde portion d'os (O₂), et
• des moyens (40) pour solidariser ensemble l'extrémité proximale émergente (12) de la première broche (10) et l'extrémité proximale émergente (22) de la seconde broche (20)
**caractérisé par le fait que** ladite l'extrémité proximale (12) de la première broche (10) est réalisée de façon qu'elle émerge de ladite fracture (F_{rc}) pour se positionner en regard de la seconde portion d'os (O₂), et que cette dite extrémité proximale émergente (12) fasse, d'une part avec l'extrémité distale (11) de ladite première broche un coude (13) d'angle β déterminé non nul, et d'autre part avec l'axe longitudinal (1) un angle γ non nul égal à α + β.

2. Système selon la revendication 1, **caractérisé par le fait qu'**il comporte en outre des moyens (30) pour guider, par rapport à l'extrémité proximale émergente (12) de la première broche (10), l'implantation de l'extrémité distale (21) de la seconde broche dans la partie corticale de la seconde portion d'os (O₂).

3. Système selon la revendication 2, **caractérisé par le fait que** les moyens (30) pour guider, par rapport à l'extrémité proximale émergente (12) de la première broche (10), l'implantation de l'extrémité distale (21) de la seconde broche dans la partie corticale de la seconde portion d'os (O₂), sont agencés de façon que la seconde broche fasse, avec l'axe longitudinal (1), un angle sensiblement égal à l'angle γ et qu'elle soit sensiblement parallèle à l'extrémité proximale émergente (12) de la première broche (10).

4. Système selon l'une des revendications 2 et 3, **caractérisé par le fait que** les moyens (30) pour guider l'implantation de l'extrémité distale de la seconde broche (20) dans la partie corticale de la seconde portion d'os (O₂) sont constitués d'une pièce guide (33) dans laquelle sont réalisées deux percées traversantes (31, 32) ayant des sections transversales respectivement complémentaires de celles des extrémités proximales émergentes (12, 22) des première et seconde broches (10, 20).

5. Système selon la revendication 4, **caractérisé par le fait que** les moyens (40) pour solidariser ensemble l'extrémité proximale émergente (12) de la première broche (10) et l'extrémité proximale émergente (22) de la seconde broche rectiligne (20) sont constitués **par le fait que** ladite pièce guide (33) est réalisée en un matériau apte à être déformé plastiquement de façon qu'elle soit apte à être sertie sur les deux extrémités proximales émergentes (12, 22) des première et seconde broches (10, 20) quand celles-ci sont respectivement positionnées dans les percées traversantes (31, 32).

6. Système selon l'une des revendications 1 à 5, **caractérisé par le fait qu'**il comporte des moyens pour tordre la première broche (10) qui est à l'origine rectiligne, de façon que son extrémité proximale émergente (12) forme avec son extrémité distale (11) ledit angle β.

7. Système selon la revendication 6, **caractérisé par le fait que** lesdits moyens pour tordre la première broche (10) à l'origine rectiligne comportent un rapporteur et une pince de pliage.

8. Système selon la revendication 7, **caractérisé par le fait que** ledit rapporteur comporte un élément apte à se fixer sur ladite première broche avant qu'elle soit tordue et sur lequel est apte à être repérée la valeur de l'angle β, de façon à pouvoir tordre ladite première broche dans n'importe quelle position de celle-ci par rapport à la première portion d'os (O₁) pour que sa partie proximale émergente (12) fasse, avec sa partie distale, ledit angle β.

9. Système selon la revendication 8, quand elle dépend de la revendication 4, **caractérisé par le fait que** les axes des deux percées traversantes (31, 32) sont sensiblement parallèles.

10. Système selon l'une des revendications précédentes, **caractérisé par le fait qu'**il comporte au moins un second ensemble, les première et seconde broches du second ensemble étant non colinéaires par rapport aux première et seconde broches (10, 20) du premier ensemble, les deux dits points (2) de la ligne de fracture pour les deux ensembles étant non confondus.

11. Système selon l'une des revendications 1 à 10, **caractérisé par le fait que** ledit angle α est sensiblement égal à 45°.

12. Système selon la revendication 11, **caractérisé par le fait que** ledit angle β est sensiblement égal à 45° de façon que l'angle γ soit sensiblement égal à 90°.
